(19) Europäisches Patentamt
European Patent Office
Office européen des brevets

(11) Publication number: 0 342 823 A1

(12) # EUROPEAN PATENT APPLICATION

(21) Application number: 89304517.9

(22) Date of filing: 04.05.89

(51) Int. Cl.4: C07D 207/448 , C07D 207/452

(30) Priority: 20.05.88 GB 8811985

(43) Date of publication of application: 23.11.89 Bulletin 89/47

(84) Designated Contracting States: BE DE ES FR GB IT NL

(71) Applicant: BP Chemicals Limited
Belgrave House 76 Buckingham Palace Road
London, SW1W 0SU(GB)

(72) Inventor: Lancaster, Michael
BP Chemicals Limited Sully
South Glamorgan CF6 2YU(GB)

(74) Representative: Krishnan, Suryanarayana Kalyana et al
BP INTERNATIONAL LIMITED Patents & Agreements Division Chertsey Road
Sunbury-on-Thames Middlesex TW16 7LN(GB)

(54) **Synthesis of bisimides.**

(57) This invention relates to a process for producing bisimides of unsaturated cyclic dianhydrides by cyclodehydration of the corresponding bisamic acid using acetic anhydride and a base component.

The latter is a combination of (a) anhydrous sodium acetate and (b) a salt selected from acetate, chloride, bromide, nitrate or sulphate of potassium, sodium chloride; the chloride or sulphate of Mg; and ferric sulphate.

The bisimides are useful cross-linking agents for elastomers.

EP 0 342 823 A1

Xerox Copy Centre

## SYNTHESIS OF BISIMIDES

This invention relates to a process for the synthesis of bisimides of unsaturated cyclic dianhydrides especially bismaleimides in the presence of sodium acetate catalysed by metal salts.

Bisimides of unsaturated cyclic dianhydrides such as bismaleimides are useful crosslinking agents for elastomers and in the copolymerisation of vinyl monomers such as styrene. For instance copolymers of bismaleimides with other monomers result in products of high thermal stability. Bisimides are usually prepared by reacting initially the unsaturated cyclic anhydride with a diamine to form the corresponding bisamic acid which is subsequently cyclodehydrated using acetic anhydride and a basic salt such as sodium acetate to form the bisimide. Such procedures for producing 2,4-bis(maleimido) toluene are described in DT-A-2127025 (Techno-Chemie GmbH), US-A-2444536 (DuPont) and US-A-3127414 (DuPont).

USP 4154737 (DuPont) describes such a process to have serious disadvantages due to the product isolation stages requiring large amounts of water to precipitate the end product and the conversion of the expensive dimethylformamide (DMF) solvent by acetic acid used in the reaction into wasteful dimethyl acetamide and formic acid. This DuPont patent recommends the use of a combination of salts of magnesium, lithium, manganese or cobalt which are soluble in the liquid reaction medium and a cocatalyst which is a tertiary amine having a pKa value of at least 10 determined in aqueous solution at 25° C.

This combination used instead of sodium acetate is said to mitigate the problems of the latter.

US-A-3839358 (Rhone-Poulenc) describes a process for preparing bismaleimides by using in the cyclodehydration step bismaleamic acid, a liquid lower carboxylic acid anhydride, a tertiary amine and a catalyst which is a nickel salt or a divalent nickel complex. Similar processes are also described in US-A-3975401 and US-A-3960887 both to Rhone-Poulenc.

It has now been found that in the case of the relatively more difficult reactions the cyclodehydration of the bisamic acid can be achieved rapidly and in high yield in the presence of an anhydride and an alkali metal acetate by using a catalyst which is free from amino groups.

Accordingly, the present invention is a process for producing bisimides of unsaturated cyclic dianhydrides by cyclodehydration of the corresponding bisamic acid using acetic anhydride and a base component characterised in that the base component is free of amino compounds and comprises: (a) an anhydrous sodium acetate and (b) a catalytic amount of a salt selected from the acetate, chloride, bromide, nitrate or sulphate salt of potassium; sodium chloride; the chloride or sulphate salt of magnesium; and ferric sulphate.

A typical example of the bisamic acid to be dehydrated is that derivable from maleic anhydride and can be represented by the generic formula:

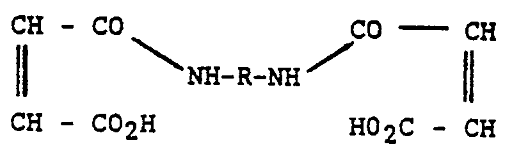

(I)

wherein R is selected from a linear or branched chain $C_2$-$C_{12}$ alkylene group; a cycloalkylene group having six carbon atoms in the ring; an arylene group; and di- or poly-alkylene or a di- or poly-arylene group in which the alkylene or arylene groups are respectively bridged by one or more groups selected from:
$-CR_1R_2-$, wherein $R_1$ and $R_2$ may be the same or different groups selected from H, an alkyl, an aryl or an alkaryl group;
$-SO_2-$;
-O-;
$-NR_3-$ wherein $R_3$ is an alkyl, aryl or an alkaryl group;
and a $-C(CF_3)_2-$ group.

Whilst the above shows bismaleamic acid as a typical example of the bisamic acid, other bisamic acids derivable from corresponding anhydrides such as citraconic and nadic anhydrides instead of maleic anhydride can also be cyclodehydrated by the present process.

The bisamic acid to be cyclodehydrated can be produced by conventional reactions well known to those skilled in the art. For instance, bismaleamic acid can be produced by reacting an appropriate primary amine with maleic anhydride at ambient temperature in the presence of a solvent such as e.g. chloroform, dimethyl formamide, acetone or toluene. Thus

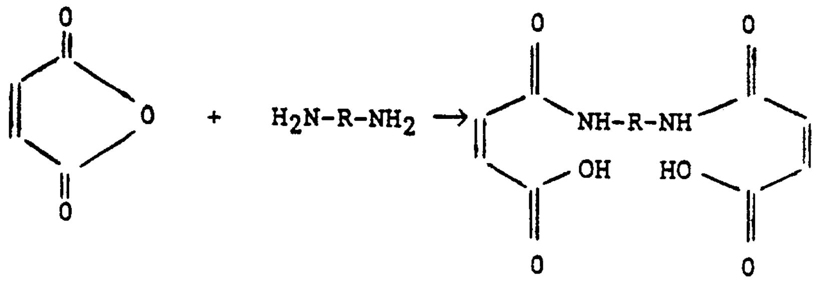

(I)

The bismaleamic acid so formed can be cyclodehydrated to the corresponding bismaleimide as represented below according to the process of the present invention:

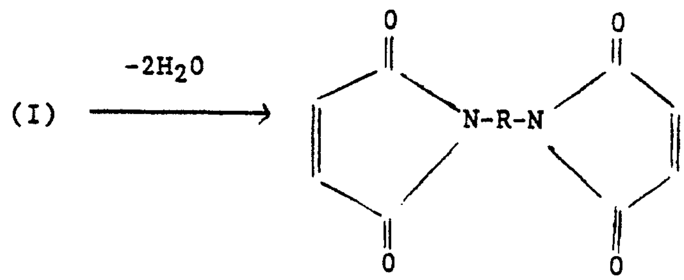


(II)

The cyclodehydration stage according to the present invention is carried out using acetic anhydride and a base component comprising anhydrous sodium acetate and a catalytic amount of an inorganic salt defined above.

By "catalytic amount" is meant here and throughout the specification that the inorganic salt is present in an amount of at least 0.5% w/w based on the bisamic acid, preferably from 0.5-2% w/w of the bisamic acid to be cyclodehydrated.

The inorganic salt in the base component can be used in the hydrated or anhydrous form. By hydrated is meant that the salt is used with its inherent water of crystallisation intact.

The anhydrous sodium acetate is suitably present in the base component in an amount of at least 8% w/w based on the bisamic acid, preferably from 8-15% w/w of the bisamic acid to be cyclodehydrated.

The mole ratio of acetic anhydride to the bisamic acid to be cyclodehydrated is suitably from 2-4 moles of the anhydride, preferably from 2.7-3.2 moles of acetic anhydride per mole of the bisamic acid.

The cyclodehydration is suitably carried out in a solvent medium. The solvent chosen should be such that the reactants and base components are substantially soluble therein. Polar, aprotic solvents such as dimethyl sulphoxide, dimethyl formamide and N-methyl pyrrolidone are preferred.

The cyclodehydration reaction is suitably carried out at a temperature from 35-70°C, preferably from 45-60°C.

The reaction is usually complete within a duration of 150 minutes, in most cases within 100 minutes.

The reaction mixture is thereafter cooled to room temperature and diluted with water. The bisimide, which is insoluble in water, is precipitated. This product is then filtered, washed and dried.

The present invention is further illustrated with reference to the following Examples.

Example 1

Toluenediamine (79.5g) dissolved in dimethylsulphoxide (79.5g) was added over 1h, at ambient temperature, to a stirred solution of maleic anhydride (130.5g) in dimethylsulphoxide (130.5g). After stirring for an additional 1/2h sodium acetate (21.3g) and catalyst (2.1g, 1% w/w based on the bismaleamic acid formed) were added. Acetic anhydride (191g) was added to the reaction mixture over approximately 1h, temperature being controlled between 40 and 55°C. After stirring for 1h at 50 C the mixture is cooled to room temperature and water (245g) added over 1h. The product was filtered, washed with water and a 1/1 mix of acetone/methanol and dried. Yield of the toluenediamine bismaleimide product (TDA-BMI) was 150g

(83%), with a purity 96% by high pressure liquid chromatography (HPLC).

The procedure of Example 1 above was repeated using different inorganic salts as catalysts in 1% w/w concentration based on the bismaleamic acid to be cyclodehydrated. The results are shown in the Table below.

| Effect of Catalysts on TDA-BMI Yield After 100 Min | | |
|---|---|---|
| Examples | Catalyst (1% w/w) | % Yield TDA-BMI |
| | None* | 64 |
| 2 | Potassium Chloride | 84 |
| 3 | Potassium Bromide | 86 |
| 4 | Potassium Sulphate | 82 |
| 5 | Potassium Nitrate | 78 |
| 6 | Potassium Acetate | 79 |
| 7 | Sodium Chloride | 75 |
| 8 | Magnesium Chloride | 71 |
| 9 | Magnesium Sulphate | 74 |
| 10 | Ferric Sulphate | 80 |

*Comparative Test not according to the invention.
TDA-BMI (Toluenediamine bismaleimide).

Example 2

Methylenedianiline (29.7g) dissolved in dimethylsulphoxide (79.5g) was added over half an hour at ambient temperature, to a stirred solution of maleic anhydride (30.9g) in dimethylsulphoxide (30.9g). After stirring for an additional half an hour sodium acetate (6g) and potassium chloride (0.6g) were added. Acetic anhydride (48.6g) was added dropwise over 40 minutes, temperature being controlled between 40 and 55 °C. The reaction was stirred at 50 °C under nitrogen and monitored by HPLC. Maximum conversion was achieved after 40 minutes. Product was cooled to 30 °C and water ($100cm^3$) added. The product was filtered, washed with water and 1/1 mix of acetone/methanol. 41.2g (75%) of product were obtained with a purity of 87% by HPLC, mp 146-149 °C.

Comparative Test - not according to the invention

A similar experiment to Example 2 was carried out in the absence of potassium chloride. Maximum conversion was achieved after 70 minutes. The product 40.6g (74%) was obtained in only 71% purity, mp 121-129 °C.

**Claims**

1. A process for producing bisimides of unsaturated cyclic dianhydrides by cyclodehydration of the corresponding bisamic acid using acetic anhydride and a base component characterised in that the base component is free of amino compounds and comprises: (a) an anhydrous sodium acetate and (b) a catalytic amount of a salt selected from the acetate, chloride, bromide, nitrate or sulphate salt of potassium; sodium chloride; the chloride or sulphate salt of magnesium; and ferric sulphate.

2. A process according to claim 1 wherein the bisamic acid to be dehydrated is that derivable from maleic anhydride and is represented by the generic formula:

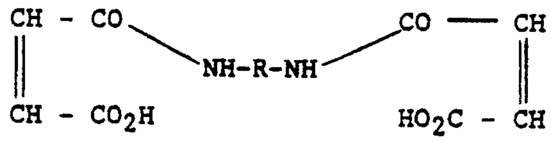

(I)

wherein R is selected from a linear or branched chain $C_2$-$C_{12}$ alkylene group; a cycloalkylene group having six carbon atoms in the ring; an arylene group; and a di- or poly-alkylene or a di- or poly-arylene group in which the alkylene or arylene groups are respectively bridged by one or more groups selected from:

$-CR_1R_2-$, wherein $R_1$ and $R_2$ may be the same or different groups selected from H, an alkyl, an aryl or an alkaryl group;

$-SO_2-$;

-O-;

$-NR_3-$ wherein $R_3$ is an alkyl, aryl or an alkaryl group;

and a $-C(CF_3)_2-$ group.

3. A process according to claim 1 or 2 wherein the inorganic salt is present in an amount from 0.5-2%w/w of the bisamic acid to be cyclodehydrated.

4. A process according to any one of the preceding claims wherein the sodium acetate is present in an anhydrous form.

5. A process according to claim 4 wherein the sodium acetate is present in the base component in an amount of at least 8%w/w based on the basamic acid to be cyclodehydrated.

6. A process according to any one of the preceding claims wherein the mole ratio of acetic anhydride to the bisamic acid to be cyclodehydrated is from 2-4 moles of anhydride per mole of bisamic acid.

7. A process according to any one of the preceding claims wherein the cyclodehydration is carried out in the presence of a polar, aprotic solvent in which both the reactants and base components are substantially soluble.

8. A process according to claim 7 wherein the solvent is dimethyl sulphoxide, formamide or N-methyl-pyrrolidone.

9. A process according to any one of the preceding claims wherein the cyclodehydration reactions is carried out at a temperature from 35-70 °C.

European Patent Office

# EUROPEAN SEARCH REPORT

Application Number

EP 89 30 4517

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | US-A-3 975 401 (RHONE-POULENC)<br>* Claim 1 *<br>--- | 1 | C 07 D 207/448<br>C 07 D 207/452 |
| A,D | US-A-3 127 414 (DU PONT DE NEMOURS)<br>* Claim 1 *<br>----- | 1 | |

TECHNICAL FIELDS SEARCHED (Int. Cl.4)

C 07 D 207/00

The present search report has been drawn up for all claims

| Place of search | Date of completion of the search | Examiner |
|---|---|---|
| THE HAGUE | 14-06-1989 | CASADO Y MARTIN DE MERCA |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)